# FASCICULE DE BREVET EUROPEEN

(11) **EP 0 678 745 B1**
(45) Date de publication et mention de la délivrance du brevet: **22.07.1998**
(21) Numéro de dépôt: 95490016.3
(22) Date de dépôt: 18.04.1995
(51) Int. Cl.: G01N 33/80, B01L 3/00

(54) **Dispositif et procédé d'analyse immunologique**
Vorrichtung und Verfahren zur immunologischen Analyse
Method and apparatus for immunological analysis

(30) Priorité: 22.04.1994 FR 9405123
(43) Date de publication de la demande: 25.10.1995
(73) Titulaire: SCIBIEX (SARL), F-59160 Capinghem (FR)
(72) Inventeur: Chateau, Sophie, F-80540 Camon (FR)
(74) Mandataire: Hénnion, Jean-Claude

(56) Documents cités:
- EP-A- 0 054 087
- EP-A- 0 104 881
- EP-A- 0 305 337
- EP-A- 0 363 510
- EP-A- 0 454 509
- WO-A-91/08491
- WO-A-93/03374
- DE-U- 9 314 161

## Description

La présente invention concerne un dispositif perfectionné pour analyse immunologique, notamment immunohématologique , mettant en oeuvre une chambre de réaction à phase solide. Elle concerne plus particulièrement un dispositif se présentant sous la forme d'un kit de deux éléments distincts, l'un constituant l'élément d'incubation et l'autre l'élément de réaction. La présente invention concerne également un procédé spécialement conçu pour la mise en oeuvre du dispositif précité.

Les techniques de recherche d'anticorps, mises en oeuvre au cours du suivi des transfusions de sang, ont fait récemment l'objet d'une évolution importante par rapport aux techniques traditionnelles en tube ou sur plaque.

Une première technique dite en phase solide, consiste à fixer de façon stable des hématies-tests sur le fond de cuvettes, profilées en U, réalisées en matière plastique. Ces cuvettes sont associées sous forme de plaque de microtitration qui en comportent 96 ou encore sous forme de barrettes qui en comportent 8 ou 12.

Selon la technique proposée par la firme Immucor, il s'agit de rechercher dans le sérum d'un patient les anticorps qui pourraient être dirigés spécifiquement contre certains antigènes présents sur les hématies-tests immobilisées, c'est-à-dire fixés sur la matière plastique constituant le fond de la cuvette. Cette recherche se fait par une première étape d'incubation du sérum dans la cuvette ainsi sensibilisée, puis par une opération de lavage, en général répétée cinq fois, qui permet d'éliminer les anticorps non fixés, et enfin par l'addition d'une suspension d'un indicateur constitué d'hématies coatées avec des antiglobulines humaines. Grâce à une centrifugation, l'indicateur vient au contact du fond de la cuvette ; si des anticorps présents dans le sérum ont été retenus par les hématies-tests immobilisées sur ledit fond , ceci provoque l'adhérence de l'indicateur et se traduit par une image homogène sur l'ensemble du fond de la cuvette. Cette image s'interprète comme une réaction positive. Si par contre aucun anticorps ne s'est fixé, l'indicateur n'adhère pas aux hématies immobilisées sur l'ensemble du fond de la cuvette et se regroupe au centre de celui-ci provoquant l'image d'une accumulation localisée. Cette image s'interprète comme une réaction négative.

Dans la technique Immucor les plaques de microtitration sont livrées à l'état desséché. Soit le fond de la cuvette est sensibilisé avec des colorants et c'est l'utilisateur qui va préparer sa couche sensible d'hématies-tests. Soit la couche sensible d'hématies est préalablement préparée et livrée sous forme lyophilisée.

Selon une variante de cette technique en phase solide, proposée par la firme Biotest, le fond de la cuvette est recouvert de globulines, les plaques de microtitration ou barrettes étant livrées desséchées.

Dans ce cas l'utilisateur va incuber dans la cuvette le mélange constitué du sérum du malade et des hématies-tests livrées en suspension. Après l'incubation, l'utilisateur doit laver in situ les hématies pour éliminer les protéines libres du mélange. Ensuite, il ajoute une antiglobuline liquide et par centrifugation on obtient la réaction des hématies, des anticorps et des antiglobulines avec le fond de la cuvette recouvert des globulines.

La technique Immucor ne prend en compte que les anticorps du type IgG, tandis que la technique Biotest permet en principe de mettre en évidence toute classe d'immunoglobuline. Cependant cette dernière est considérée comme difficile à mettre en oeuvre.

Dans les deux cas, l'obligation de dessécher les substances que l'on fixe sur le fond de la cuvette en matière plastique est une contrainte importante au stade de la fabrication des plaques de microtitration ou des barrettes. De plus la dessiccation ou la lyophilisation implique l'addition de substances protectrices qui cependant ne peuvent éviter totalement l'altération des activités biologiques des produits fixés.

Une seconde technique est dite TAC , c'est-à-dire Techniques d'Agglutinations en Colonnes. Elle est proposée par les firmes Diamed et Ortho. Ces techniques en colonnes utilisent des microtubes associés sous forme de cartes ou de cassettes comportant en général six colonnes, chacune étant constituée par un tube contenant soit du gel de Sephadex® ou assimilé soit des microbilles de verre en suspension dans un diluant spécifique. Le haut de la colonne s'évase pour former une chambre d'incubation apte à recevoir les sérums et hématies pour la recherche d'anticorps. Le gel ou le diluant contient du sérum de Coombs, à base d'antiglobulines humaines ou encore un diluant neutre dans le cas de techniques utilisant des enzymes protéolytiques. Pour les groupages sanguins, le gel ou le diluant spécifique contient des sérums-tests et le principe de fonctionnement et de regroupement est identique à celui des recherches d'anticorps.

De manière générale, cette seconde technique TAC s'est révélée plus sensible que les techniques traditionnelles en tube et plus commode que les techniques en phase solide notamment, pour les réactions à l'antiglobuline, elles dispensent l'utilisateur des phases de lavage nécessaires à l'élimination des protéines non fixées.

En effet le gel ou le diluant, dans le cas des microbilles de verre, joue un rôle de gradient de densité, laissant passer les hématies et non les protéines libres. Cette seconde technique fait donc partie des techniques dites de microtitration. Le gel ou les microbilles ont la capacité de retenir les agglutinats au sommet de la colonne et de laisser passer, sous l'influence de la force centrifuge, les hématies non agglutinées. Les agglutinats sont créés soit au niveau de la chambre d'incubation soit au contact des réactifs contenus dans le gel ou dans le diluant.

Contrairement à la première technique dite de phase solide, les réactifs contenus dans les colonnes sont à l'état liquide. Une languette métallique plastifiée et soudée sur le haut de la cassette en assure l'étanchéité ; elle doit être enlevée au moment de l'utilisation. La technique TAC présente cependant des inconvénients. En effet les colonnes de gel ou de microbilles peuvent être facilement endommagées lors du transport, étant particulièrement sensibles aux chocs. De plus on observe fréquemment des éclaboussures qui détruisent ou altèrent la qualité des résultats et qui peuvent être responsable de contaminations croisées au moment d'ouverture des cassettes par enlèvement de la languette de protection. Lors du transport des gouttes de réactif peuvent être projetées sur la languette et contaminer les autres colonnes, lors de l'enlèvement de celle-ci. De plus on observe fréquemment des phénomènes de condensation au niveau de la languette métallique, ce qui modifie la concentration des produits contenus dans la colonne et par voie de conséquence altère leurs caractéristiques réactionnelles.

Le but de la présente invention est de proposer un dispositif pour analyse immunologique qui pallie les inconvénients précités des deux techniques actuellement les plus performantes.

Ce but est parfaitement obtenu grâce au dispositif de l'invention qui, de manière caractéristique, se présente sous forme d'un kit composé de deux éléments distincts, destinés à être assemblés et à coopérer lors de l'analyse immunologique proprement dite le premier élément dit d'incubation comporte au moins un réceptacle apte à recevoir comme contenu un mélange composé d'un produit analysé et d'un produit test ; le second élément dit de réaction comporte au moins une cuvette qui est fermée par une membrane d'étanchéité et sur le fond de laquelle est fixée une couche réactionnellement sensible. De plus le dispositif de l'invention comporte, associé au premier et au second éléments, d'une part des moyens d'assemblage desdits éléments et d'autre part des moyens de perforation et d'injection qui sont aptes à réaliser d'abord la perforation de la membrane puis l'injection du contenu du réceptacle à travers ladite membrane perforée, une fois que les deux éléments sont assemblés.

Certes le document EP 0 054 087 décrit un dispositif pour analyse qui est composé de deux éléments qui comportent des moyens d'assemblage et des moyens de perforations et qui sont destinés à être assemblés lors de l'analyse proprement dite. Mais la structure des deux éléments et leur fonction respective diffèrent totalement de l'objet de l'invention. En particulier, les deux éléments n'étaient pas distincts, il ne s'agit pas d'un kit ; le premier élément du document EP 0 054 087 ne comporte pas de réceptacle d'incubation ; le second élément du document EP 0 054 087 comporte une cavité ouverte, sur laquelle il n'est pas prévu de fixer une couche réactionnellement sensible, au contraire la cuvette du second élément du dispositif de l'invention est fermée par une membrane d'étanchéité et a un fond sur lequel est fixé une couche réactionnellement sensible.

Ainsi il y a une totale indépendance entre les deux éléments au stade de la fabrication du dispositif. Ce n'est que lors de l'assemblage que les deux éléments coopèrent en vue de l'analyse immunologique. Cette séparation en deux éléments distincts présente comme autre avantage de minimiser les volumes de produits spécifiques à conserver avec précaution, notamment dans des conditions de température déterminée, puisque seul le second élément de réaction est dans ce cas, alors que le premier élément d'incubation peut être stocké sans précaution particulière.

Les moyens de perforation et d'injection consistent en une tubulure effilée , formée à partir du fond du réceptacle de l'élément d'incubation et tournée vers l'extérieur ; l'orifice intérieur de ladite tubulure a un diamètre tel que le contenu du réceptacle ne peut diffuser que sous l'action d'une force centrifuge déterminée. De plus la tubulure effilée du réceptacle est disposée en sorte de perforer la membrane d'étanchéité lors de l'assemblage des deux éléments.

Compte-tenu de la dimension particulière de l'orifice intérieur de la tubulure, le contenu du réceptacle ne peut s'écouler lors du stockage de celui-ci, alors qu'il va pouvoir grâce à une simple centrifugation passer du réceptacle de l'élément d'incubation jusqu'à la cuvette de l'élément de réaction. Le diamètre le plus étroit de la tubulure sera par exemple compris entre 0,5 et 0,7 mm.

On comprend que les moyens d'assemblage associés aux deux éléments doivent permettre le positionnement l'un par rapport à l'autre des deux éléments de telle sorte que la tubulure effilée puisse perforer la membrane d'étanchéité et pénétrer dans la cuvette une fois l'opération d'assemblage réalisée.

De préférence les moyens d'assemblage consistent :
a) en ce qui concerne l'élément d'incubation, en des pattes de fixation qui s'étendent en dessous du niveau du réceptacle , sur une hauteur suffisante pour constituer des pieds de protection pour la tubulure effilée,
b) en ce qui concerne l'élément de réaction, en des évidements dans lesquels peuvent se clipser les pattes de fixation.

Ainsi, grâce à cette disposition particulière les pattes de fixation assurent non seulement la stabilité de l'élément d'incubation lors de son stockage mais également la protection des tubulures effilées prolongeant les réceptacles.

Avantageusement la cuvette contient un constituant qui est apte à maintenir humide la couche sensible. Il peut s'agir notamment d'une solution protéique, sucrée ou d'un gel . Ceci permet d'éviter les opérations de déshydratation après la fixation de la couche réactionnellement sensible sur le fond de la cuvette et donc de garantir une meilleure stabilité des substances biologiquement active ainsi fixées.

De préférence le constituant apte à maintenir humide la couche sensible possède des propriétés chromatographiques en sorte que, sous l'action de la force centrifuge, les éléments corpusculaires diffusent vers le fond de la cuvette plus rapidement que les protéines.

Selon un mode de réalisation, l'élément de réaction est constitué :
a) d'un ruban en plastique mince, dans lequel sont préformée une pluralité de cuvettes à fond en V ou en U,
b) d'un socle support ayant une pluralité de parties en creux et évidées pour recevoir, chacune une cuvette dont le fond est accessible par transparence pour l'examen des résultats de l'analyse,
c) et d'une membrane d'étanchéité, recouvrant toutes les cuvettes et fixée sur le socle support en sorte d'obturer de manière étanche toutes les cuvettes.

Selon un autre mode de réalisation, l'élément de réaction ne comporterait plus de ruban préformé mais seulement un socle support, par exemple en plastique injecté, dans lequel les parties en creux ne seraient pas évidées mais présenteraient un fond accessible par transparence, constituant des cuvettes à fond en V ou en U.

Ledit socle support peut notamment consister en une plaque traditionnelle de microtitration à 96 cuvettes, à raison de 8 rangées de 12 cuvettes.

C'est un autre objet de l'invention que de proposer un procédé d'analyse immunologique mettant en oeuvre le dispositif précité dont le fond des réceptacles est muni d'une tubulure effilée.

De manière caractéristique le procédé de l'invention comprend les étapes qui consistent à:
a) assembler un élément d'incubation, qui contient dans son réceptacle un mélange de composants parmi lesquels le produit à analyser et le produit test et dont le fond perforé est prolongé extérieurement par une tubulure effilée dont le diamètre intérieur est tel qu'il ne permet pas la diffusion du mélange pendant l'assemblage et l'incubation, avec un élément de réaction comportant une cuvette, fermée par une membrane d'étanchéité et sur le fond de laquelle est fixée une couche réactionnellement sensible, ledit assemblage étant réalisé de manière à ce que la tubulure effilée perfore la membrane d'étanchéité et pénètre dans la cuvette,
b) incuber ledit mélange,
c) centrifuger les deux éléments assemblés à une vitesse suffisante pour que le mélange diffuse depuis le réceptacle jusque dans la cuvette à travers la tubulure effilée.

De préférence, la couche réactionnellement sensible étant recouverte d'un constituant hydratant à propriété chromatographique, le procédé comprend une étape supplémentaire de centrifugation à une vitesse suffisante pour obtenir la diffusion différenciée des composants du mélange à travers le constituant hydratant.

La présente invention sera mieux comprise à la lecture de la description qui va être faite d'un exemple de réalisation du dispositif d'analyse immunologique et de sa mise en oeuvre, illustré par le dessin annexé dans lequel :
- la figure 1 est une représentation schématique en coupe des deux éléments d'incubation et de réaction, superposés avant assemblage,
- la figure 2 représente les mêmes deux éléments qu'à la figure 1 une fois assemblés,
- la figure 3 est une représentation schématique en perspective d'un kit de l'invention à 96 cuvettes.

Le kit 1 d'analyse immunologique selon l'invention se compose de deux éléments distincts 2 et 3 dont les circuits de fabrication et de stockage peuvent être indépendants mais qui sont destinés à être assemblés et à coopérer lors de l'analyse immunologique.

Le premier élément 2, dénommé élément d'incubation, comporte au moins un réceptacle 4, mais plus généralement une pluralité de réceptacles. Chaque réceptacle 4 est constitué d'une cuvette à fond en forme de U, ayant une contenance d'environ 100 à 200 µl. Le fond 4a du réceptacle se prolonge par une tubulure effilée 5 dont le diamètre intérieur est de l'ordre de 0,5 mm. Cette dimension de diamètre ne permet pas l'écoulement par simple gravité naturelle des liquides qui sont contenus dans le réceptacle 4.

L'élément d'incubation comporte également des pattes de fixation 6. Lesdites pattes 6 prolongent le corps 7 de l'élément d'incubation 2 sur une hauteur suffisante pour que l'extrémité libre 8 de la tubulure effilée 5 soit au-dessus du plan DD passant par les extrémités libres 9 desdites pattes 6. Ainsi lorsque l'élément d'incubation 2 est stocké, il repose sur les pattes de fixation 6 sans risque de choc pour les extrémités libres 8 des tubulures effilées 5.

L'élément de réaction 3 comporte au moins une cuvette 10 ou plus généralement une pluralité de cuvettes 10 en nombre identique au nombre de réceptacles 4 de l'élément d'incubation 2 correspondant.

Sur le fond 10a de la cuvette 10, en forme de V ou de U, est fixé une couche réactionnellement sensible.

La cuvette 10 est fermée de manière étanche par une membrane 11, par exemple une languette métallique et plastifiée.

La cuvette 10 est disposée dans un évidement, sensiblement conique, qui est creusé dans un support 12, à travers l'intégralité de celui-ci de manière à ce que le fond 10a de la cuvette 10 soit visible sur la face inférieure 12a du support 12 rendant accessible par transparence les résultats de l'analyse immunologique.

Le support 12 comporte également des trous d'encliquetage 13 qui sont conformés pour accueillir les pattes de fixation 6 de l'élément d'incubation 2 ou comporte une saignée en cas d'emboîtage. La profondeur des trous d'encliquetage 13 est déterminée en sorte qu'une fois l'encliquetage réalisé l'extrémité 8 de la tubulure effilée 5 soit dans la zone intérieure de la cuvette 10.

S'agissant d'un dispositif modulaire du type de celui qui est décrit dans le document FR 2 655 151, les deux éléments 2, 3 sont réalisés à partir de blocs de plastique, confèrant à l'ensemble sa rigidité et sa facilité de prise en main, l'élément d'incubation 2 étant en plastique cristal transparent tandis que l'élément de réaction 3 peut être dans la même matière ou en plastique injecté coloré.

Dans ce cas, les réceptacles 4 font partie intégrante dudit bloc, tandis que les tubulures effilées 5 sont des éléments rapportés par rapport audit bloc.

En ce qui concerne les cuvettes 10 de l'élément de réaction 3, elles sont préformées dans une feuille fine de matière plastique, par exemple de polycarbonate et ont une contenance d'environ 100 à 250 µl. Chaque cuvette 10 a reçu un traitement préalable de sensibilisation ou coating dans sa partie base correspondant au fond 10a. Ce traitement de sensibilisation est destiné à réaliser la fixation sur le matériau plastique constitutif du fond 10a de la cuvette 10 d'une couche réactionnellement sensible . Celle-ci est bien sûr fonction du type d'analyse immunologique qui est envisagé . Il s'agit notamment d'antigènes ou d'anticorps.

De préférence on ajoute, après le traitement de sensibilisation, dans la cuvette 10 un liquide macromoléculaire ou un gel du type Sephadex® qui permet de maintenir la couche sensible à l'état humide.

La feuille plastique, préformée, est insérée dans le bloc plastique 12.

Comme prévu dans le document FR 2 655 151 ledit bloc peut recevoir sur sa surface extérieure des identifications sous forme de code à barres ou de pistes magnétiques, destinées à permettre un traitement automatisé du dispositif de l'invention.

La mise en oeuvre du dispositif 1 est réalisée de la manière suivante. L'opérateur introduit dans les réceptacles 4 de l'élément d'incubation 2 un mélange constitué du produit à analyser et du produit test. S'agissant par exemple de la recherche d'anticorps irréguliers en immunohématologie mettant en oeuvre un dispositif 1 à quatre cuvettes 10, on introduit dans les réceptacles 4, 30µl de sérum du malade et 30µl d'hématies-tests en suspension à 0,2 %. En pratique on dispose dans les trois premiers réceptacles trois hématies-tests différents tandis que le quatrième réceptacle sert de contrôle.

Les cuvettes 10 de l'élément de réaction 3 ont été préalablement sensibilisées avec des anticorps antiglobulines humaines.

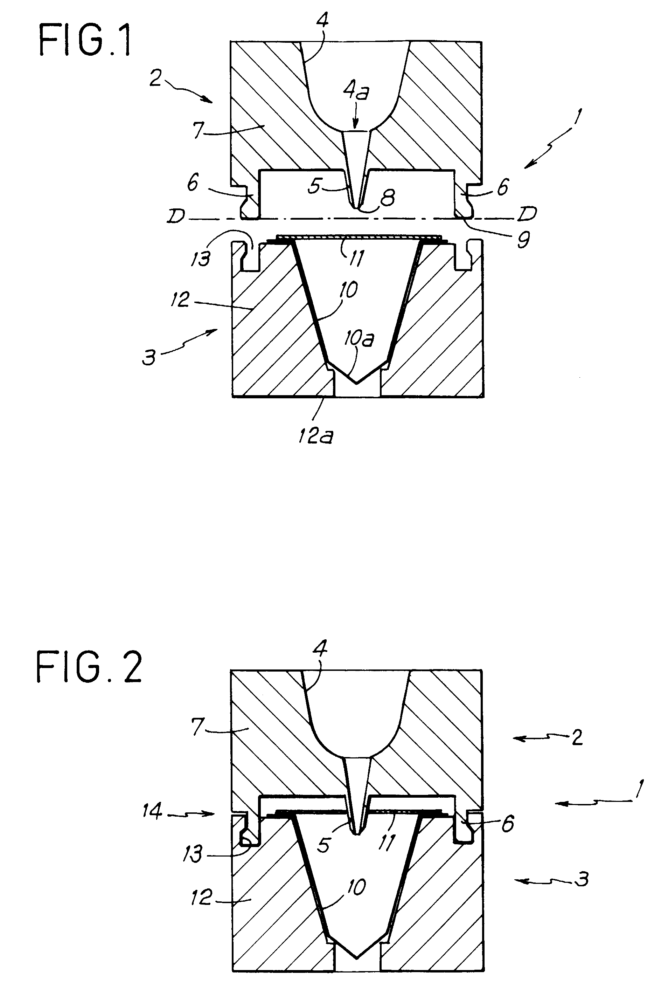

On dispose l'élément d'incubation 2 au-dessus de l'élément de réaction 3 de manière à faire pénétrer les pattes de fixation 6 dans les trous 13, jusqu'à clipsage desdites pattes 6 dans lesdits trous 13. Ce faisant, les extrémités 8 des tubulures effilées 5 sont venues en contact avec la surface extérieure de la feuille d'étanchéité 11 puis l'ont progressivement perforée jusqu'à ce que lesdites extrémités 8 pénètrent à l'intérieur des cuvettes 10 comme cela est illustré à la figure 2.

Le module 14 constitué des deux éléments d'incubation 2 et de réaction 3 ainsi assemblés est disposé dans un incubateur, par exemple pendant 15 minutes à 37 ° C ou encore dans une armoire ventilée.

A la suite de cette incubation, le module assemblé 14 est placé dans une centrifugeuse. La vitesse de centrifugation est d'abord déterminée pour que le mélange sérum et hématies-tests soit repoussé à travers la tubulure effilée 5 par l'action de la force centrifuge jusqu'à être injecté à l'intérieur de la cuvette 10. La force est par exemple de 80 g.

Dans le cas où la cuvette 10 contient également un constituant hydratant, par exemple un gel Sephadex®, on applique ensuite une vitesse plus rapide de centrifugation qui permet de faire descendre, sous l'action de la force centrifuge, les hématies-tests pour venir au contact du fond 10a en V de la cuvette 10. Il s'agit par exemple d'une vitesse permettant d'appliquer une force de 150g pendant une minute.

Dans le cas où une liaison s'établit entre les hématies-tests et l'antiglobuline fixée sur le fond 10a de la cuvette on observe une répartition homogène sur tout le fond 10a et la réaction est positive.

Dans le cas où aucune adhérence n'est observée, les hématies-tests se concentrent dans la partie centrale du fond 10a et la réaction est négative.

On comprend, à la lecture de la description qui vient d'être faite, que grâce au système à tubulures effilées le dispositif de l'invention permet le transfert du mélange disposé dans l'élément d'incubation jusqu'à la cuvette de réaction sans risque de contamination croisée et sans risque d'éliminer les éventuelles parties de réactifs qui seraient condensées ou éclaboussées lors du transport, notamment sous la languette de protection.

Etant donné que la membrane d'étanchéité, qui fait office de languette de protection, reste à demeure, on évite ainsi les risques de déchirements partiels de la languette lors de l'enlèvement de celle-ci comme cela pouvait se produire dans les dispositifs antérieurs.

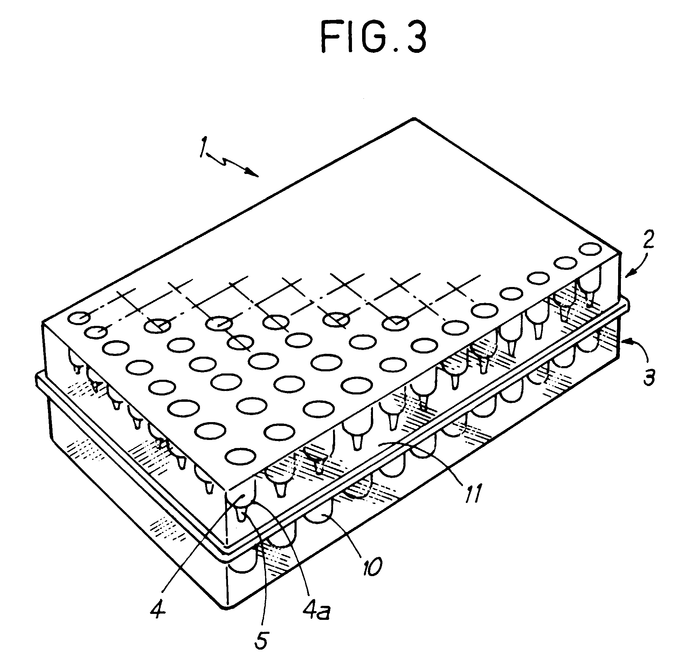

Dans l'exemple décrit, il s'agissait de modules comportant un nombre faible de cuvettes de réaction, par exemple quatre. Il pourrait bien sûr s'agir de modules présentant un nombre plus important de cuvettes, pouvant constituer des barrettes ou plaques de type microtitration, comme cela est illustré à la figure 3 avec 96 réceptacles et 96 cuvettes disposées à raison de huit rangées de 12 cuvettes.

De préférence le traitement de sensibilisation du fond 10a des cuvettes 10 est basé sur l'adsorption électrostatique des protéines sur le matériau plastique constitutif de la cuvette.

De préférence également il s'agit de fixer sur le fond de ladite cuvette une antiglobuline à réaction IgG ou polyvalente, et non de constituer un tapis d'hématies comme dans la technique proposée par Immucor. Ainsi le procédé de l'invention évite les lavages, ce qui constitue un gain de temps appréciable et conduit à une plus grande stabilité et reproductibilité. En effet les lavages peuvent conduire éventuellement à l'arrachement des hématies fixées et donc à des réactions faussement positives par adsorption de l'indicateur ou encore à des réactions d'aspect douteux. Ces lavages constituent dans les techniques précédentes, un écueil essentiel pour des utilisateurs non expérimentés.

Il est à noter que le gel qui est mis en oeuvre dans la cuvette 10 de réaction a une fonction hydratante et protectrice de la couche sensible du fond 10a de la cuvette 10. Ce gel ne contient jamais de réactif, contrairement à la technique TAC. Le but visé par la présence de ce gel ou d'autres liquides macromoléculaires est d'éviter la mise en oeuvre des procédures de déshydratation, après sensibilisation du fond 10a et donc de garantir une meilleure stabilité des substances biologiquement actives.

Avantageusement ce gel comporte 1 % d'albumine bovine pour saturer les sites qui ne seraient pas coatés. Le gel Sephadex® peut éventuellement être remplacé par une solution d'albumine à 30 % ou une autre macromolécule comportant une fonction protectrice et hydratante.

Au moment de la centrifugation, il se produit une chromatographie à travers le gel. Celui-ci va en effet ralentir la diffusion des protéines libres dans le milieu réactionnel et va laisser descendre plus rapidement les hématies.

Contrairement à ce qui se passe dans la technique TAC, les hématies ne sont, selon le présent procédé, jamais agglutinées pour que la réaction d'adhérence sur le fond 10a de la cuvette 10 puisse avoir lieu.

La présente invention n'est pas limitée au mode de réalisation qui vient d'être décrit à titre d'exemple non exhaustif . En particulier les moyens d'assemblage qui ont été proposés peuvent prendre des formes multiples pour autant que soit respectée la fonction première qui leur est impartie à savoir de constituer grâce à cette assemblage un module compact et manipulable entre les deux éléments d'incubation et de réaction et d'obtenir, par l'intermédiaire des moyens de perforation et d'injection, la capacité de transférer le contenu du réceptacle d'incubation dans la cuvette de réaction. Les moyens d'assemblage peuvent notamment fonctionner par emboîtement avec une partie mâle et une partie femelle disposées respectivement sur l'élément d'incubation et l'élément de réaction ou réciproquement.

## Revendications

1. Dispositif pour analyse immunologique composé de deux éléments comportant des moyens d'assemblage et des moyens de perforation, les deux dits éléments étant destinés à être assemblés lors de l'analyse immunologique proprement dite, caractérisé en ce qu'il se présente sous la forme d'un kit composé de deux éléments indépendants, le premier élément dit d'incubation (2) comportant au moins un réceptacle (4) apte à recevoir comme contenu un mélange composé d'un produit à analyser et d'un produit test et le second élément dit de réaction (3) comportant au moins une cuvette (10) qui est fermée par une membrane d'étanchéité (11) et sur le fond (10a) de laquelle est fixée une couche réactionnellement sensible, en ce que les moyens de perforation consistent en une tubulure effilée (5) , formée à partir du fond (4a) du réceptacle (4) de l'élément d'incubation (2) et tournée vers l'extérieur, l'orifice intérieur de ladite tubulure (5) ayant un diamètre tel que le contenu du réceptacle ne peut diffuser que sous l'action d'une force centrifuge déterminée, et en ce que ladite tubulure effilée (5) est disposée en sorte de perforer la membrane d'étanchéité (11) lors de l'assemblage des deux éléments (2, 3)

2. Dispositif selon la revendication 1 caractérisé en ce que les moyens d'assemblage consistent :
a) en ce qui concerne l'élément d'incubation (2), en des pattes de fixation (6) qui s'étendent en dessous du niveau du réceptacle (4), sur une hauteur suffisante pour constituer des pieds de protection pour la tubulure effilée (5),
b) en ce qui concerne l'élément de réaction (3), en des évidements (13) dans lesquels peuvent se clipser les pattes de fixation (6).

3. Dispositif selon l'une des revendications 1 à 2 caractérisé en ce que la cuvette (10) contient un constituant qui est apte à maintenir humide la couche sensible, notamment une solution protéique, sucrée ou un gel .

4. Dispositif selon la revendication 3 caractérisé en ce que le constituant apte à maintenir humide la couche sensible possède des propriétés chromatographiques en sorte que, sous l'action de la force centrifuge, les éléments corpusculaires diffusent vers le fond (10a) de la cuvette (10) plus rapidement que les protéines.

5. Dispositif selon l'une des revendications 1 à 4 caractérisé en ce que l'élément de réaction (3) est constitué :
a) d'un ruban en plastique mince, dans lequel sont préformées une pluralité de cuvettes à fond en V ou en U,
b) d'un socle support (12) ayant une pluralité de parties en creux et évidées pour recevoir, chacune une cuvette dont le fond est accessible par transparence pour l'examen des résultats de l'analyse ,
c) et d'une membrane d'étanchéité (11), recouvrant toutes les cuvettes (10) et fixée sur le socle support en sorte d'obturer de manière étanche toutes les cuvettes.

6. Dispositif selon l'une des revendications 1 à 4 caractérisé en ce que l'élément de réaction (3) est constitué :
a) d'un socle support ayant une pluralité de parties en creux, non évidées, constituant des cuvettes à fond en V ou en U, ledit fond étant accessible par transparence pour l'examen des résultats de l'analyse,
b) et d'une membrane d'étanchéité, recouvrant toutes les parties en creux et fixée sur le socle support en sorte d'obturer de manière étanche toutes les cuvettes.

7. Dispositif selon la revendication 6 caractérisé en ce que l'élément d'incubation et l'élément de réaction comportent respectivement 96 réceptacles et 96 cuvettes, à raison de huit rangées de douze.

8. Procédé d'analyse immunologique mettant en oeuvre le dispositif selon la revendication 1, caractérisé en ce qu'il comprend les étapes qui consistent à :
a) assembler un élément d'incubation (2), qui contient dans son réceptacle (4) un mélange de composants parmi lesquels le produit à analyser et le produit test et dont le fond (4a) est prolongé extérieurement par une tubulure effilée (5) dont le diamètre intérieur est tel qu'il ne permet pas la diffusion du mélange pendant l'assemblage et l'incubation, avec un élément de réaction (3) comportant une cuvette (10), fermée par une membrane d'étanchéité (11) et sur le fond (10a) de laquelle est fixée une couche réactionnellement sensible, ledit assemblage étant réalisé de manière à ce que la tubulure effilée (5) perfore la membrane d'étanchéité (11) et pénètre dans la cuvette (10),
b) incuber ledit mélange,
c) centrifuger les deux éléments (2, 3) assemblés à une vitesse suffisante pour que le mélange diffuse depuis le réceptacle (4) jusque dans la cuvette (10) à travers la tubulure effilée (5).

9. Procédé selon la revendication 8 caractérisé en ce que la couche réactionnellement sensible étant recouverte d'un constituant hydratant à propriété chromatographique, il comprend une étape supplémentaire de centrifugation à une vitesse suffisante pour obtenir la diffusion différenciée des composants du mélange à travers le constituant hydratant.

## Patentansprüche

1. Vorrichtung zur immunologischen Analyse, die aus zwei Elementen besteht, welche Mittel zur Montage und Mittel zur Perforation aufweisen, wobei die beiden Elemente dazu bestimmt sind, bei der eigentlichen immunologischen Analyse zusammengefügt zu werden,
dadurch **gekennzeichnet**, daß
sie in Form eines Kits vorliegt, der aus zwei unabhängigen Elementen besteht, wobei das erste Inkubationselement (2) mindestens einen Behälter (4) aufweist, der als Inhalt ein Gemisch aufnehmen kann,
das aus einem zu analysierenden Produkt und einem Testprodukt zusammengesetzt ist, und das zweite Reaktionselement (3) mindestens eine Küvette (10) aufweist, die mit einer Dichtmembran (11) verschlossen ist und an deren Boden (10a) eine reaktionsempfindliche Schicht angebracht ist,
die Mittel zur Perforation aus einem sich verjüngenden Stutzen (5) bestehen, der ausgehend vom Boden (4a) des Behälters (4) des Inkubationselements (2) gebildet und nach außen gerichtet ist, wobei die innere Öffnung des Stutzens (5) einen solchen Durchmesser aufweist, daß der Inhalt des Behälters nur unter Einwirkung einer vorbestimmten Zentrifugalkraft hindurchtreten kann und der sich verjüngende Stutzen (5) so angeordnet ist,
daß er die Dichtmembran (11) beim Zusammenfügen der beiden Elemente (2, 3) durchbohrt.

2. Vorrichtung nach Anspruch 1, dadurch **gekennzeichnet**, daß die Mittel zur Montage bestehen aus:
a) bezüglich des Inkubationselements (2) aus Befestigungsfüßen (6), die sich unter dem Behälter (4) auf eine Höhe erstrecken, die ausreichend ist, damit sie Schutzelemente für den sich verjüngenden Stutzen (5) darstellen, und
b) bezüglich des Reaktionselements (3) Aussparungen (13), in die die Befestigungsfüße (6) einrasten können.

3. Vorrichtung nach einem der Ansprüche 1 bis 2, dadurch **gekennzeichnet**, daß die Küvette (10) einen Bestandteil enthält, der dazu befähigt ist, die empfindliche Schicht feucht zu halten, insbesondere eine Protein-Lösung, Zuckerlösung oder ein Gel.

4. Vorrichtung nach Anspruch 3, dadurch **gekennzeichnet**, daß der Bestandteil, der befähigt ist, die empfindliche Schicht feucht zu halten, chromatographische Eigenschaften aufweist, so daß die Partikel unter der Einwirkung der Zentrifugalkraft schneller in Richtung Boden (10a) der Küvette (10) diffundieren als die Proteine.

5. Vorrichtung nach einem der Ansprüche 1 bis 4, dadurch **gekennzeichnet,** daß das Reaktionselement (3) besteht aus:
a) einem dünnen Kunststoffband, in dem eine Vielzahl von Küvetten mit V- cder U-förmigem Boden vorgeformt sind,
b) einem Trägersockel (12) mit mehreren Aussparungen, welche ausgehöhlt wurden, um jeweils eine Küvette aufzunehmen, deren Boden aufgrund seiner Transparenz zur Beurteilung der Analyseergebnisse zugänglich ist, und
c) einer Dichtmembran (11), die alle Küvetten (10) abdeckt und so an dem Trägersockel befestigt ist, daß alle Küvetten dicht verschlossen sind.

6. Vorrichtung nach einem der Ansprüche 1 bis 4, dadurch **gekennzeichnet,** daß das Reaktionselement (3) besteht aus:
a) einem Trägersockel mit mehreren Aussparungen, die nicht ausgehöhlt wurden und Küvetten mit V- oder U-förmigem Boden darstellen, wobei der Boden aufgrund seiner Transparenz zur Beurteilung der Analyseergebnisse zugänglich ist, und
b) einer Dichtmembran, die alle Aussparungen abdeckt und an dem Trägersockel so befestigt ist, daß alle KÜvetten dicht verschlossen sind.

7. Vorrichtung nach Anspruch 6, dadurch **gekennzeichnet**, daß das Inkubationselement bzw. das Reaktionselement 96 Behälter bzw. 96 Küvetten in acht 12er-Reihen aufweisen.

8. Verfahren zur immunologischen Analyse unter Verwendung der Vorrichtung nach Anspruch 1, dadurch **gekennzeichnet**, daß es die folgenden Schritte umfaßt:
a) Verbinden eines Inkubationselements (2), das in seinem Behälter (4) ein Gemisch von Komponenten, darunter das zu analysierende Frodukt und das Testprodukt, enthält und dessen Boden (4a) sich über einen sich verjüngenden Stutzen (5) nach außen erstreckt, dessen innerer Durchmesser so ausgeführt ist, daß das Gemisch bei der Montage und der Inkubation nicht hindurchtreten kann, mit einem Reaktionselement (3), das eine Küvette (10) aufweist, die mit einer Dichtmembran (11) verschlossen ist und an deren Boden (10a) eine reaktionsempfindliche Schicht befestigt ist, wobei die Montage so durchgeführt wird, daß der sich verjüngende Stutzen (5) die Dichtmembran (11) durchbohrt und in die Küvette (10) eindringt,
b) Inkubation des Gemisches, und
c) Zentrifugieren der beiden zusammengefügten Elemente (2, 3) bei einer Geschwindigkeit, die ausreichend ist, damit das Gemisch vcm Behälter (4) durch den sich verjüngenden Stutzen (5) in die Küvette (10) eintritt.

9. Verfahren nach Anspruch 8, dadurch **gekennzeichnet**, daß die reaktionsempfindliche Schicht mit einem hydratisierenden Bestandteil mit chromatographischen Eigenschaften bedeckt ist und das Verfahren einen zusätzlichen Zentrifugierschritt bei einer Geschwindigkeit aufweist, die ausreichend ist, um eine unterschiedliche Diffusion der Bestandteile des Gemisches durch den hydratisierenden Bestandteil zu erzielen.

## Claims

1. Apparatus for immunological analysis, the apparatus comprising two elements including assembly means and perforation means, said two elements being designed to be assembled together during immunological analysis proper, characterised in that it is in the form of a kit comprising two independent elements, the first element being an incubation element (2) that includes at least one receptacle (4) suitable for receiving as its contents a mixture made up of a substance to be analysed and a test substance, and the second element (3) constituting a reaction element including at least one well (10) which is closed by a sealing membrane (11) and which has a bottom (10a) on which a reactionally sensitive layer is fixed, in that the perforation means are a tapering tube (5) extending from the bottom (4a) of the receptacle (4) of the incubation element (2) and pointing outwards, the inside orifice of said tube (5) having a diameter such that the contents of the receptacle can diffuse therethrough only under drive from a determined centrifugal force, and in that said tapering tube (5) is disposed so as to perforate the sealing membrane (11) when the two elements (2, 3) are assembled together.

2. Apparatus according to claim 1, characterised in that the assembly means consist:
a) for the incubation element (2), in fixing tabs (6) which project beneath the bottom of the receptacle (4) far enough to constitute legs that protect the tapering tube (5); and
b) for the reaction element (3) in recesses (13) in which said fixing tabs (6) can be snap-fastened.

3. Apparatus according to one of claims 1 to 2, characterised in that the well (10) contains a substance suitable for keeping the sensitive layer moist, in particular a protein solution, a sugar solution, or a gel.

4. Apparatus according to claim 3, characterised in that the substance suitable for keeping the sensitive layer moist possesses chromatographic properties such that, under the action of centrifugal force, the corpuscular elements diffuse towards the bottom (10a) of the well (10) more quickly than do the proteins.

5. Apparatus according to one of claims 1 to 4, characterised in that the reaction element (3) is constituted:
a) by a thin plastics tape in which a plurality of V- or U-bottomed wells are formed;
b) by a support base (12) having a plurality of open-ended recessed portions each serving to receive one well whose bottom is accessible by transparency for examining the results of the analysis; and
c) by a sealing membrane (11) covering all of the wells (10) and fixed to the supporting base in such a manner as to close all of the wells in sealed manner.

6. Apparatus according to one of claims 1 to 4, characterised in that the reaction element (3) is constituted:
a) by a support base having a plurality of closed-ended recessed portions constituting V- or U-bottomed wells, said bottom being accessible by transparency for examining the results of the analysis; and
b) a sealing membrane covering all of the hollow portions and fixed to the supporting base in such a manner as to close all of the wells in sealed manner.

7. Apparatus according to claim 6, characterised in that the incubation element and the reaction element comprise respectively 96 receptacles and 96 wells, organised as eight rows of twelve each.

8. A method of immunological analysis implementing the apparatus according to claim 1, characterised in that it comprises the following steps:
a) assembling an incubation element (2) that contains in its receptacle (4) a mixture of substances including the substance to be analysed and the test substance, and whose bottom (4a) is extended externally by a tapering tube (5) whose inside diameter is such as to prevent the mixture from passing therethrough during assembly and incubation, with a reaction element (3) that includes a well (10) closed by a sealing membrane (11) and having a bottom (10a) on which there is fixed a reactionally sensitive layer, said assembly being made in such a manner that the tapering tube (5) perforates the sealing membrane (11) and penetrates into the well (10);
b) incubating said mixture; and
c) centrifuging the two assembled-together elements (2, 3) at a speed sufficient to cause the mixture to pass from the receptacle (4) into the well (10) via the tapering tube (5).

9. A method according to claim 8, characterised in that the reactionally sensitive layer is covered by a hydrating substance having chromatographical properties, the method including an additional step of centrifuging at a speed sufficient to obtain differentiated diffusion of the component substances of the mixture through the hydrating substance.
